# EUROPÄISCHE PATENTSCHRIFT

(11) **EP 1 660 030 B1**
(45) Veröffentlichungstag und Bekanntmachung des Hinweises auf die Patenterteilung: **27.11.2013**
(21) Anmeldenummer: 04739687.4
(22) Anmeldetag: 08.06.2004
(51) Int. Cl.: A61K 8/37, A61K 8/44, A61Q 17/04

(54) **MISCHUNG BESTEHEND AUS EINEM UV-A- UND EINEM UV-B-FILTER**
MIXTURE CONSISTING OF UV-A AND UV-B FILTERS
MELANGE CONSTITUE D'UN FILTRE POUR UV-A ET D'UN FILTRE POUR UV-B

(30) Priorität: 24.06.2003 DE 10328547
(43) Veröffentlichungstag der Anmeldung: 31.05.2006
(73) Patentinhaber: BASF SE, 67056 Ludwigshafen (DE)
(72) Erfinder: ANDRE, Valerie, 67063 Ludwigshafen (DE); WÜNSCH, Thomas, 67346 Speyer (DE); HEIDENFELDER, Thomas, 67125 Dannstadt (DE)
(86) Internationale Anmeldenummer: PCT/EP2004/006158
(87) Internationale Veröffentlichungsnummer: WO 2004/112740

(56) Entgegenhaltungen:
- DE-A- 10 063 946
- DE-A- 10 254 335
- DE-A- 10 260 876
- DE-C- 957 162
- BASF: 'Uvinul® A Plus', [Online] Gefunden im Internet: <URL:http://www.basf-chemtrade.de/images/st ories/broschueren/PCI/090324_uvinul_a_plus- final.pdf>

## Beschreibung

>

Die vorliegende Erfindung betrifft eine Mischung in flüssiger Form mit einer Viskosität von 300 - 500 mPa*s bestehend aus einem UV-A-Filter der Formel I und einem UV-B-Filter der Formel II, 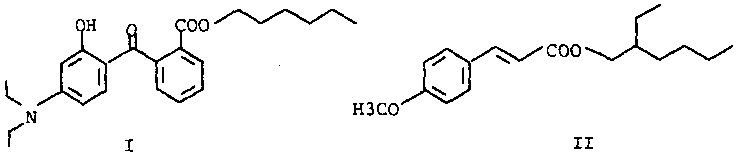 worin die Mischung aus 30 bis 50 Gew.-% des UV-A-Filters I und 70 bis 50 Gew.-% des UV-B-Filters II besteht, und ein Auskristallisieren des UV-A-Filters der Formel I verhindert wird.

Die Verwendung aminosubstituierter Hydroxybenzophenone als photostabile UV-Filter in kosmetischen und dermatologischen Zubereitungen wurde erstmals in der EP 1 046 391 A2 beschrieben.

Kosmetische Zubereitungen enthaltend Mischungen von aminosubstituierten Hydroxybenzophenonen mit im UV-A-, im UV-B-Bereich und über beide Bereiche absorbierende Verbindungen sind u.a. beschrieben in EP 1 133 980 A2, EP 1 240 984 A2, EP 1 291 009 sowie in WO 03/039507.

Ein für Sonnenschutzmittel bevorzugt verwendetes aminosubstituiertes Hydroxybenzophenon ist die eingangs erwähnte Verbindung der Formel I, die unter der Handelsbezeichnung Uvinul^{□} A Plus von der Fa. BASF hergestellt und vermarktet wird.

Die Herstellung und Aufreinigung von Uvinul^{®} A Plus, beschrieben in DE-A-10221805, erfolgt dabei u.a. durch Behandlung mit einem Adsorbens und einer anschließenden destillativen Abtrennung des Lösungsmittels. Das so erhaltene Endprodukt kann dabei nach der Destillation als Schmelze verpack werden.

Dabei kann es passieren, dass der Ester während der Lagerung aus der Schmelze auskristallisiert und das Produkt nur durch wiederholtes Aufschmelzen aus dem Gebinde entnommen werden kann. Für viele Hersteller kosmetischer Zubereitungen, die nicht über die notwendigen Apparaturen zum Aufschmelzen fester Substanzen verfügen, ergeben sich daraus erhebliche anwendungstechnische Probleme.

Es war daher Aufgabe der vorliegenden Erfindung, Uvinul^{®} A Plus in einer Form bereitzustellen, mit der ein unerwünschtes Auskristallisieren dieses UV-A-Filters aus seiner Schmelze verhindert wird.

Diese Aufgabe wurde gelöst durch das Bereitstellen einer Mischung bestehend aus einem UV-A-Filter der Formel I und einem UV-B-Filter der Formel II.

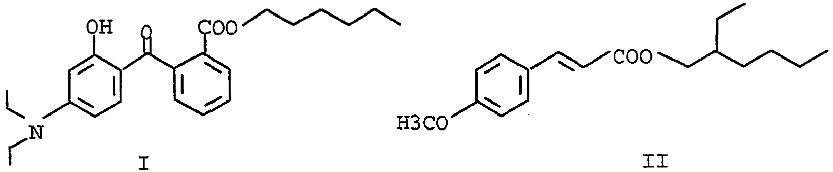

p-Methoxyzimtsäure-2-ethylhexylester der Formel II stellt ein farbloses Öl dar und gehört mit zu den am häufigsten verwendeten UV-B-Filtern in kosmetischen und dermatologischen Zubereitungen. Unter dem Namen Uvinul^{®} MC 80 ist Verbindung II von Fa. BASF erhältlich.

Die erfindungsgemäße Mischung bietet für den Hersteller von Lichtschutzmittelzubereitungen den Vorteil, dass ein unerwünschtes Auskristallisieren von Uvinul^{®} A Plus, insbesondere in den Verpackungsgebinden verhindert wird und die damit verbundenen Probleme beim Entleeren dieser Gebinde nicht mehr auftreten. Somit ist es nicht mehr notwendig, Uvinul^{®} A Plus haltige Gebinde, beispielsweise Flaschen oder Container bei Temperaturen > 20°C zu lagern.

Ferner stellt die erfindungsgemäße Mischung einen photostabilen Breitbandfilter dar, der es ermöglicht, sowohl UV-A- als auch UV-B-Strahlen zu absorbieren.

Die oben genannte Mischung ist dadurch gekennzeichnet, dass sie aus 30 bis 50 Gew.-%, besonders bevorzugt 35 bis 45 Gew.-% des UV-A-Filters I und 70 bis 50 Gew.-%, besonders bevorzugt 65 bis 55 Gew.-% des UV-B-Filters II besteht.

Die Mischung zeichnet sich dadurch aus, dass sie in flüssiger Form mit einem ausgezeichneten Fließverhalten vorliegt.

Die Viskositäten der erfindungsgemäßen Mischungen liegen im Bereich von 300 bis 500 mPa*s.

Die Herstellung dieses Gemisches erfolgt in an sich bekannter Weise durch einfaches Vermischen der Verbindungen I und II, wobei die Reihenfolge der Zugabe der Einzeikomponenten nicht von Bedeutung ist.

Gegenstand der vorliegenden Erfindung ist auch die Verwendung einer Mischung, definiert gemäß einem der Ansprüche 1 bis 3 zur Herstellung von kosmetischen und pharmazeutischen Zubereitungen zum Schutz der menschlichen Haut und menschlicher Haare gegen UV-Strahlung.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wässriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Als Emulsionen kommen u.a. auch O/W-Makroemulsionen, O/W-Mikroemulsionen W/O/W- oder O/W/O-Emulsionen mit in dispergierter Form vorliegenden aminosubstituierten Hydroxybenzophenonen der Formel I in Frage, wobei die Emulsionen durch Phaseninversionstechnologie, gemäß DE-A-197 26 121 erhältlich sind.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polycrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxy-benzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkoimmission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Ein zusätzlicher Gehalt an Antioxidantien ist im allgemeinen bevorzugt. So können als günstige Antioxidantien alle für kosmetische und/oder dermatologische Anwendungen geeigneten oder gebräuchlichen Antioxidantien verwendet werden.

Vorteilhafterweise werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. β-Carotin, Lycopin) und deren Derivate, Chlorogensäure und deren Derivate, Liponsäure und deren Derivate (z.B. Dihydroliponsäure), Aurothioglucose, Propylthiouracil und andere Thiote (z.B. Thiorodoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäuure und deren Derivate (Ester, Ether, Peptide, Lipide, Nukleotide, Nukleoside und Salze) sowie Sulfoximinverbindungen (z.B. Buthioninsulfoximine, Homocysteinsulfoximine, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Biliburin, Biliverdin, EDTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und deren Derivate (z.B. Ascorbylpalmitat, Mg-Ascorbylphosphat, Ascorbylacetat), Tocopherol und Derivate (z.B. Vitamin-E-Acetat, Tocotrienol), Vitamin A und Derivate (Vitamin-A-Palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid).

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Sofern Vitamin A und/oder dessen Derivate bzw. Carotinoide das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Der Gesamtanteil der Hilfs- und Zusatzstoffe kann 1 bis 80, vorzugsweise 6 bis 40 Gew.-% und der nicht wässrige Anteil ("Aktivsubstanz") 20 bis 80, vorzugsweise 30 bis 70 Gew.-%- bezogen auf die Mittel - betragen. Die Herstellung der Mittel kann in an sich bekannter Weise, d.h. beispielsweise durch Heiß-, Kalt-, Heiß-Heiß/Kalt-bzw. PIT-Emulgierung erfolgen.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wässrige Lotionen.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Als UV-Filtersubstanzen, die in Kombination mit der erfindungsgemäß zu verwendenden Mischung angewandt werden, kommen beliebige UV-A- und UV-B-Filter-substanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 2-Hydroxy-4-methoxy-benzophenon-5-sulfon-(Sulisobenzonum) und das Natriumsalz | 4065-45-6 |
| 12 | 3-(4'-Sulfo)benzyliden-bornan-2-on und Salze | 58030-58-6 |
| 13 | 3-Benzylidenbornan-2-on | 16087-24-8 |
| 14 | 1-(4'-Isopropylphenyl)-3-phenylpropan-1,3-dion | 63260-25-9 |
| 15 | 4-Isopropylbenzylsalicylat | 94134-93-7 |
| 16 | 2,4,6-Trianilin-(o-carbo-2'-ethylhexyl-1'-oxy)-1,3,5-triazin | 88122-99-0 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoate oder: | 134-09-8 |
| | 5-Methyl-2-(1-methylethyl)-2-aminobenzoate | |
| 21 | Glyceryl p-aminobenzoat oder: | 136-44-7 |
| | 4-Aminobenzoesäure-1-glyceryl-ester | |
| 22 | 2,2'-Dihydroxy-4-methoxybenzophenon (Dioxybenzone) | 131-53-3 |
| 23 | 2-Hydroxy-4-methoxy-4-methylbenzophenon (Mexonon) | 1641-17-4 |
| 24 | Triethanolamin Salicylat | 2174-16-5 |
| 25 | Dimethoxyphenylglyoxalsäure oder: | 4732-70-1 |
| | 3,4-dimethoxy-phenyl-glyoxal-saures Natrium | |
| 26 | 3-(4'Sulfo)benzyliden-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | 2,2'-Methylen-bis-[6(2H-benzotriazol-2-yl)-4-(1,1,3,3,-tetramethylbutyl)phenol] | 103597-45-1 |
| 30 | 2,2'-(1,4-Phenylen)-bis-1 H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |

Die erfindungsgemäßen kosmetischen und dermatologischen Zubereitungen können vorteilhafterweise außerdem anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden enthalten. Besonders bevorzugt handelt es sich um Pigmente auf der Basis TiO₂ und ZnO.

Es ist besonders vorteilhaft im Sinne der vorliegenden Erfindung, wenngleich nicht zwingend, wenn die anorganischen Pigmente in hydrophober Form vorliegen, d.h. dass sie oberflächlich wasserabweisend behandelt sind. Diese Oberflächenbehandlung kann darin bestehen, dass die Pigmente nach an sich bekannter Weise, wie in DE-A-33 14 742 beschrieben, mit einer dünnen hydrophoben Schicht versehen sind.

Zum Schutz menschlicher Haare vor UV-Strahlen können die erfindungsgemäßen Mischungen in Shampoos, Lotionen, Gelen, Haarsprays, Aerosol-Schaumcremes, Conditioner oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Mischungen zeichnen sich in der Regel durch ein besonders hohes Absorptionsvermögen im Bereich der UV-A- und UV-B-Strahlung mit scharfer Bandenstruktur aus. Weiterhin sind sie gut in kosmetischen Ölen löslich und lassen sich leicht in kosmetische Formulierungen einarbeiten. Die mit den Verbindungen I hergestellten Emulsionen zeichnen sich besonders durch ihre hohe Stabilität, die Verbindungen I selber durch ihre hohe Photostabilität aus, und die mit I hergestellten Zubereitungen durch ihr angenehmes Hautgefühl aus.

Die UV-Filterwirkung der erfindungsgemäßen Mischungen kann auch zur Stabilisierung von Wirk- und Hilfsstoffen in kosmetischen und pharmazeutischen Formulierungen ausgenutzt werden.

In den folgenden Beispielen wird die Herstellung und Verwendung der Mischung näher erläutert.

### Beispiele

### Herstellung

### Beispiel 1

### Herstellung eines Gemischs aus 40 Gew.-% 2-(4-Diethylamino-2-hydroxybenzoyl)-benzoesäure-n-hexylester und 60 Gew.-% p-Methoxyzimtsäure-2-ethylhexylester

400 g der nach DE-A-10221805 Beispiel 3 erhaltenen Schmelze von 2-(4-Diethyl-amino-2-hydroxybenzoyl)-benzoesäure-n-hexylester werden bei Raumtemperatur mit 600 g p-Methoxyzimtsäure-2-ethylhexylester versetzt und mittels eines mechanischen Rührers homogenisiert. Die Viskosität dieser Mischung betrug bei Raumtemperatur 370 mPa*s (gemessen mit Brookfield Viskosimeter bei 20 UpM).

Allgemeine Vorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

### Zubereitungen

### Beispiel 2 - Zusammensetzung für die Lippenpflege

### Massengehalt (Gew.-%)

| | |
|---|---|
| ad 100 | Eucerinum anhydricum |
| 10,00 | Glycerin |
| 10,00 | Titanium Dioxid, mikronisiert |
| 5,00 | Mischung aus Beispiel 1 |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 3 - Zusammensetzung für Sunblocker mit Mikropigmenten

### Massengehalt (Gew.-%)

| | |
|---|---|
| ad 100 | Wasser |
| 10,00 | Octyl Methoxcinnamat |
| 6,00 | PEG-7-Hydrogenated Castor Öl |
| 6,00 | Titanium Dioxid, mikronisiert |
| 5,00 | Mischung aus Beispiel 1 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 4 - Fettfreies Gel

### Massengehalt (Gew.-%)

| | |
|---|---|
| ad 100 | Wasser |
| 7,00 | Titanium Dioxid, mikronisiert |
| 5,00 | Mischung aus Beispiel 1 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 5 - Sonnencreme (LSF 20)

### Massengehalt (Gew.-%)

| | |
|---|---|
| ad 100 | Wasser |
| 8,00 | Titanium Dioxid, mikronisiert |
| 6,00 | PEG-7-Hydrogenated Castor Öl |
| 13,00 | Mischung aus Beispiel 1 |
| 6,00 | Mineral Öl |
| 5,00 | Zink Oxid |
| 5,00 | Isopropyl Palmitat |
| 0,30 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 6 - Sonnencreme wasserfest

### Massengehalt (Gew.-%)

| | |
|---|---|
| ad 100 | Wasser |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 13,00 | Mischung aus Beispiel 1 |
| 4,00 | Glycerin |
| 3,00 | Jojoba Öl |
| 2,00 | 4-Methylbenzyliden Campher |
| 2,00 | Titanium Dioxid, mikronisiert |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 7 - Sonnenmilch (LSF 6)

### Massengehalt (Gew.-%)

| | |
|---|---|
| ad 100 | Wasser |
| 10,00 | Mineral Öl |
| 6,00 | PEG-7-Hydrogenated Castor Öl |
| 5,00 | Isopropyl Palmitat |
| 8,00 | Mischung aus Beispiel 1 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Tocopheryl Acetat |
| 3,00 | Glycerin |
| 0,25 | Methylparaben |
| 0,15 | Propylparaben |
| 0,05 | Tocopherol |

## Patentansprüche

1. Mischung bestehend aus einem UV-A-Filter der Formel I und einem UV-B-Filter der Formel II.

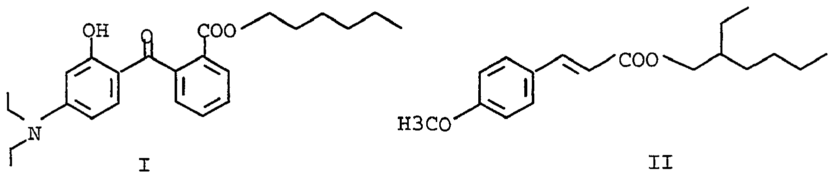

2. Mischung nach Anspruch 1 bestehend aus 30 bis 70 Gew.-% des UV-A-Filters I und 70 bis 30 Gew.-% des UV-B-Filters II.

3. Mischung nach einem der Ansprüche 1 oder 2 in flüssiger Form.

4. Verwendung einer Mischung, definiert gemäß einem der Ansprüche 1 bis 3 zur Herstellung von kosmetischen und pharmazeutischen Zubereitungen zum Schutz der menschlichen Haut und menschlicher Haare gegen UV-Strahlung.

## Claims

1. A mixture consisting of a UV-A filter of the formula I and a UV-B filter of the formula II.

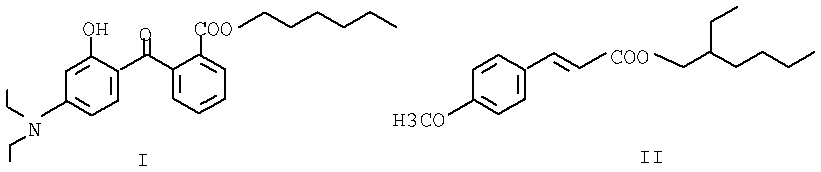

2. The mixture according to claim 1, consisting of 30 to 70% by weight of the UV-A filter I and 70 to 30% by weight of the UV-B filter II.

3. The mixture according to either of claims 1 and 2 in liquid form.

4. The use of a mixture defined according to one of claims 1 to 3 for producing cosmetic and pharmaceutical preparations for protecting human skin and human hair against UV radiation.

## Revendications

1. Mélange constitué d'un filtre UV-A de formule I et d'un filtre UV-B de formule II.

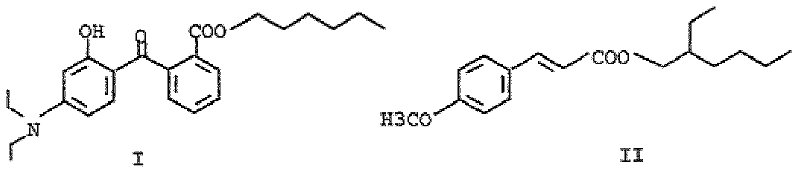

2. Mélange selon la revendication 1, constitué de 30 à 70 % en poids du filtre UV-A I et de 70 à 30 % en poids du filtre UV-B II.

3. Mélange selon la revendication 1 ou 2, sous forme liquide.

4. Utilisation d'un mélange, défini selon l'une quelconque des revendications 1 à 3, pour la fabrication de préparations cosmétiques et pharmaceutiques destinées à la protection de la peau humaine et des cheveux humains contre le rayonnement UV.
